# EUROPÄISCHE PATENTSCHRIFT

(11) **EP 3 024 836 B1**
(45) Veröffentlichungstag und Bekanntmachung des Hinweises auf die Patenterteilung: **11.07.2018**
(21) Anmeldenummer: 14741287.8
(22) Anmeldetag: 18.07.2014
(51) Int. Cl.: C07F 9/50, C07F 15/00, C07C 231/12, C07C 233/15

(54) **VERBESSERTES VERFAHREN ZUR HERSTELLUNG VON CHLORIERTEN BIPHENYLANILIDEN UND BIPHENYLANILINEN**
IMPROVED METHOD FOR THE PREPARATION OF CHLORINATED BIPHENYLANILIDES AND BIPHENYLANILINES
PROCÉDÉ AMÉLIORÉ DE FABRICATION DE BIPHÉNYLANILIDES CHLORÉS ET DE BIPHÉNYLANILINES

(30) Priorität: 23.07.2013 EP 13177583
(43) Veröffentlichungstag der Anmeldung: 01.06.2016
(73) Patentinhaber: Bayer CropScience Aktiengesellschaft, 40789 Monheim am Rhein (DE)
(72) Erfinder: DOCKNER, Michael, 50674 Köln (DE); RODEFELD, Lars, 51375 Leverkusen (DE); HEINRICH, Jens, Dietmar, 51381 Leverkusen (DE)
(74) Vertreter: BIP Patents
(86) Internationale Anmeldenummer: PCT/EP2014/065463
(87) Internationale Veröffentlichungsnummer: WO 2015/011032

(56) Entgegenhaltungen:
- EP-A1- 2 008 991
- WO-A1-2009/135598
- WO-A2-2004/052939
- US-A1- 2011 237 799
- US-B1- 6 307 087
- US-B1- 6 352 990
- WOLFE J P: "Highly active palladium catalysts for Suzuki coupling reactions", JOURNAL OF THE AMERICAN CHEMICAL SOCIETY, ACS PUBLICATIONS, US, Bd. 121, Nr. 41, 20. Oktober 1999 (1999-10-20), Seiten 9550-9561, XP002132767, ISSN: 0002-7863, DOI: 10.1021/JA992130H

## Beschreibung

Die Erfindung betrifft ein verbessertes Verfahren zur Herstellung von halogenierten Biphenylaniliden und Biphenylanilinen durch Suzuki-Kupplung von Brom- oder Jodaniliden bzw. Brom- oder Jodanilinen mit chlorierten Organoborverbindungen. Biphenylanilide und Biphenylaniline dienen als Vorstufen zur Herstellung von Pflanzenschutzmitteln mit fungizider Wirkung.

Durch Palladium katalysierte Kreuzkupplung halogenierter Aromaten der Formel (II) mit Organoborverbindungen der Formel (III) gewinnt man nach WO 2006/092429, WO 2007/138089 und WO 2009/135598 sowohl halogenierte Biphenylaniline als auch halogenierte Biphenylanilide der allgemeinen Formel (I) nach dem folgenden Schema 1. 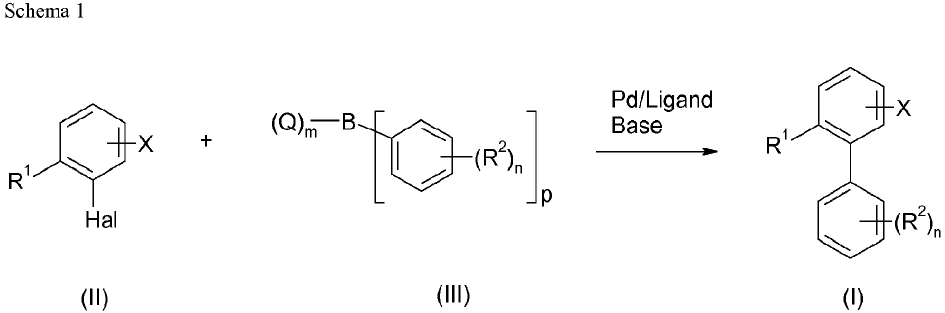 wobei die Substituenten wie folgt definiert sind:
- X: ist Wasserstoff, Fluor oder Chlor;
- Hal: ist Halogen
- R¹: ist ausgewählt aus einer geschützten Aminogruppe, NO₂, NH₂ und NHR³;
- R²: ist ausgewählt aus Cyano, Nitro, Halogen, C₁-C₆-Alkyl, C₁-C₆-Alkenyl, C₁-C₆-Alkynyl, C₁-C₆-Alkoxy, C₁-C₆-Haloalkyl, (C₁-C₆-Alkyl)Carbonyl und Phenyl;
- R³: ist ausgewählt aus Wasserstoff, -CH₂-(C=O)CH₃, C₁-C₈-Alkyl, C₁-C₈-Alkenyl, C₁-C₈-Alkynyl und C₆-C₁₈-Aryl
- n: ist ausgewählt aus 1, 2 und 3,
- p: ist ausgewählt aus 1, 2, 3 und 4 und
- Q: ist ausgewählt aus Hydroxyl, F, Cl, Br, I, C₁₋₄-Alkyl-, C₆₋₁₀-Aryl-, C₁₋₄-Alkoxy- und C₆₋₁₀-Aryloxy, C₁₋₄-Alkoxy Resten, die zusammen mit dem Boratom, an welches sie gebunden sind, einen 5- oder 6-gliedrigen Ring bilden, der mit C₁₋₄-Alkyl-Resten substituiert sein kann
und die Verbindungen der Formel (III) unterschiedliche Organoborverbindungen umfassen.

Als Liganden für die im obigen Schema dargestellte Suzuki-Reaktion kommen unsubstituierte und substituierte Trialkyl- oder Triarylphosphine zur Anwendung. Darüber hinaus eignen sich nach WO 2001/042197 auch Dialkylarylphosphine als Liganden. Die Auswahl möglicher Phosphinliganden wird von WO 2004/052939, S. 34f um Phosphine erweitert, bei denen einer der Reste am Phosphor aus einem biphenylischen Grundgerüst besteht.

Stehen in der allgemeinen Formel (II) die Substituenten
Hal für Brom oder Iod, und
X für Wasserstoff, Fluor oder Chlor, 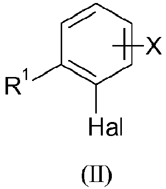 und der Substituent R² in der allgemeinen Formel (III) für Chlor, 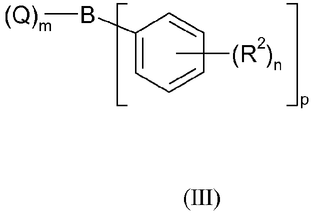 so reagiert unter den Bedingungen der Suzuki-Reaktion gebildetes, chloriertes Biphenylanilid bzw. Biphenylanilin mit Borverbindungen der allgemeinen Formel (III) weiter zu Triarylen der allgemeinen Formel (IV) und seinen höheren Homologen (Schema 2). Daraus resultiert ein Ausbeuteverlust an dem gewünschten Biphenylanilid bzw. Biphenylanilin der allgemeinen Formel (I).

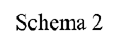

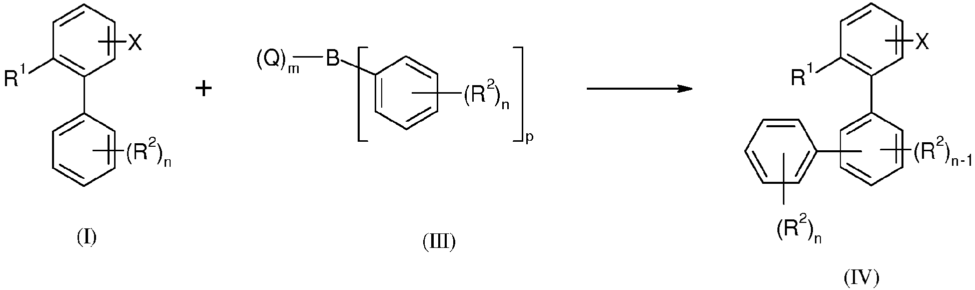

In welchem Ausmaß diese Folgereaktion stattfindet, bestimmt im Wesentlichen der in der Reaktion eingesetzte Phosphinligand.

WO 2004/052939 beschreibt die Verwendung von biphenylischen Phosphinen und Di(tert.-butyl)phenylphosphin, die die Suzuki-Reaktion von den im Vergleich zu Arylbromiden weniger reaktiven Arylchloriden mit Phenylboronsäuren selbst unter sehr milden Bedingungen katalysieren. Es ist in einem solchen Fall davon auszugehen, dass Triaryle in einem merklichen Ausmaß gebildet werden.

Durch die Einführung einer Schutzgruppe am Stickstoffatom gelingt es nach WO 2009/135598, den Anteil an Triarylen zu vermindern. In der Suzuki-Reaktion können sowohl Trialkyl- als auch Triarylphosphine eingesetzt werden.

EP2008991A1 beschreibt ein Verfahren zur Herstellung von Biarylen durch Umsetzung eines Halogenaromaten mit einer Boronsäure, einem cyclischen Boronsäureester oder einem Boronat in Gegenwart wenigstens eines Palladiumphosphin-Komplexes, wobei die Phosphin-Gruppe mit wenigstens einer verzweigten C₃₋₈-Alkyl-Gruppe substituiert ist. Dabei werden Methyl-di-(tert-butyl)phosphin und Tri(tert-butyl)phosphin als besonders bevorzugte Liganden beschrieben.

US 6,352,990 B1 beschreibt die Synthese von CRF-Rezeptor-Antagonisten. Dabei werden auch Suzuki-Reaktionen verwendet, bei denen jeweils Tetrakis(triphenylphosphin)palladium (Pd(PPh₃)₄) als Katalysator eingesetzt wird.

Aufgabe der vorliegenden Erfindung war es, einen Liganden zu finden, der Triaryle nicht oder in einem sehr geringen Maß entstehen lässt und so einen wirtschaftlicheren Zugang zu chlorierten Biphenylaniliden bzw. Biphenylanilinen ermöglicht.

Die Aufgabe wird überraschend gelöst durch ein verbessertes Verfahren zur Herstellung von chlorierten Biphenylaniliden und Biphenylanilinen der Formel (I) 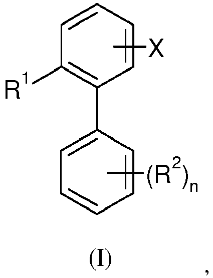 wobei die Substituenten wie folgt definiert sind:
- X: ist ausgewählt aus Wasserstoff, Fluor und Chlor;
- R¹: ist ausgewählt aus einer geschützten Aminogruppe, NO₂, NH₂ und NHR³;
- R²: ist Chlor;
- R³: ist ausgewählt aus Wasserstoff, -CH₂-(C=O)CH₃, C₁-C₈-Alkyl, C₁-C₈-Alkenyl, C₁-C₈-Alkynyl und C₆-C₁₈-Aryl.
- n: ist ausgewählt aus 1, 2 und 3,
durch Umsetzung von Halogenaromaten der allgemeinen Formel (II) 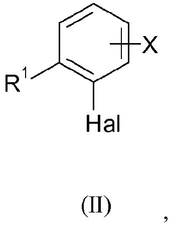 worin
- Hal: ausgewählt ist aus Brom und Jod; und X und R¹ wie oben definiert sind,
in Gegenwart einer Base und eines Palladium Katalysators ausgewählt aus der Gruppe bestehend aus
a) einem Komplex bestehend aus Palladium in der Oxidationsstufe 0 und einem Phosphinliganden der Formel (V) oder einem Salz hiervon,
b) einem Palladiumsalz in Gegenwart eines Phosphinliganden der Formel (V) oder einem Salz hiervon und
c) metallischem, gegebenenfalls auf einen Träger aufgebrachtem Palladium, in Gegenwart eines Phosphinliganden der Formel (V) oder einem Salz hiervon,
   wobei der Phosphinligand der Formel (V) wie folgt definiert ist 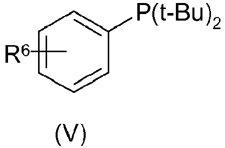 wobei
   - R⁶: ausgewählt ist aus Wasserstoff, C₁-C₄ Alkyl, C₁-C₄ Haloalkyl, Phenyl und NR⁷ und
   - R⁷: ausgewählt ist aus (C₁-C₄-Alkyl)₂
   oder ein Salz hiervon,
in einem Lösungsmittel, mit einer Organoborverbindung der Formel (III) 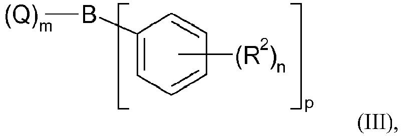 ausgewählt aus der Gruppe bestehend aus:
(i) Boronsäuren der Formel (III) in welchen
   - m: 2 ist,
   - p: 1 ist,
   - Q¹ und Q²: Hydroxylgruppen sind,
   - R² und n: wie oben definiert sind,
   oder die aus den Boronsäuren der Formel (III) gebildeten Anhydride, Dimere oder Trimere;
(ii) Boronsäurederivate der Formel (III), in welchen
   - m: 2 ist,
   - p: 1 ist,
   Q¹ und Q² jeweils unabhängig voneinander ausgewählt sind aus F, Cl, Br, I, C₁₋₄-Alkyl-, C₆₋₁₀-Aryl-, C₁₋₄-Alkoxy- und C₆₋₁₀-Aryloxy sind,
   R² und n wie oben definiert sind;
(iii) Borinsäuren der Formel (III), in welchen
   - m: 1 ist,
   - p: 2 ist,
   - Q: ausgewählt ist aus OH, F, Cl, Br, I, C₁₋₄-Alkyl-, C₆₋₁₀-Aryl-, C₁₋₄-Alkoxy- und C₆₋₁₀-Aryloxy-Resten,
   - R² und n: wie oben definiert sind;
(iv) cyclische Boronsäureester der Formel (III), in welchen
   - m: 2 ist,
   - p: 1 ist,
   - Q¹ und Q²: jeweils unabhängig voneinander ausgewählt sind aus C₁₋₄-alkoxy Resten, die zusammen mit dem Boratom, an welches sie gebunden sind, einen 5- oder 6-gliedrigen Ring bilden, der mit C₁₋₄-Alkyl-Resten substituiert sein kann,
   - R² und n: wie oben definiert sind;
(v) Boronate der Formel (III), in welchen
   - m: 3 ist,
   - p: 1 ist,
   - R² und n: wie oben definiert sind,
   - Q¹ bis Q³: jeweils unabhängig voneinander ausgewählt sind aus OH, F, Cl, Br, I, C₁₋₄-Alkyl-, C₆₋₁₀-Aryl-, C₁₋₄-Alkoxy- und C₆₋₁₀-Aryloxy-Resten,
   und worin die negative Ladung des Boronat Anions durch ein Kation kompensiert ist;
(vi) Triarylborane der Formel (III), in welchen
   - m: 0 ist,
   - p: 3 ist,
   - R² und n: wie oben definiert sind;
(vii) Tetraarylborate der Formel (III), in welchen
   - m: 0 ist,
   - p: 4 ist,
   - R² und n: wie oben definiert sind,
   und in welchen die negative Ladung des Boronat Anions durch ein Kation kompensiert ist.

Eine Ausführungsform der vorliegenden Erfindung betrifft das oben beschriebene Verfahren zur Herstellung substituierter Biphenylanilide der Formel (I) 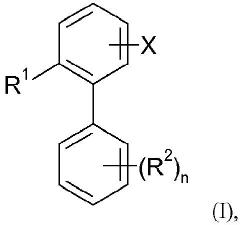 worin
- X: ausgewählt ist aus Wasserstoff, Fluor und Chlor;
- R¹: ausgewählt ist aus -NH(CO)R³, -N=CR⁴R⁵, NO₂, NH₂, und NHR³;
- R²: Chlor ist;
- R³, R⁴, R⁵: unabhängig voneinander ausgewählt sind aus Wasserstoff, -CH₂-(C=O)CH₃, C₁-C₈-Alkyl, C₁-C₈-Alkenyl, C₁-C₈-Alkynyl und C₆-C₁₈-Aryl; oder
- R⁴, R⁵: zusammen mit dem Kohlenstoffatom, an das sie gebunden sind einen 5- oder 6-gliedrigen Ring bilden können, der 1, 2 oder 3 Heteroatome ausgewählt aus N, O, und S enthält;
- n: ausgewählt ist aus 1, 2 und 3.

Gemäß einer Ausführungsform der vorliegenden Erfindung wird als Phosphinligand eine Verbindung der Formel (V-i) eingesetzt 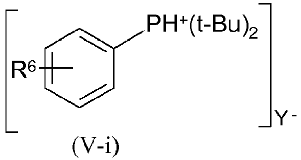 wobei
- R⁶: ausgewählt ist aus Wasserstoff, C₁-C₄ Alkyl, C₁-C₄ Haloalkyl, Phenyl und NR⁷ und
- R⁷: ausgewählt ist aus (C₁-C₄-Alkyl)₂ und
- Y: ausgewählt ist aus der Gruppe bestehend aus BF₄⁻, Perchlorat und Hydrogensulfat.

Überraschenderweise wurde gefunden, dass der Einsatz von Phosphinliganden der allgemeinen Formel (V) 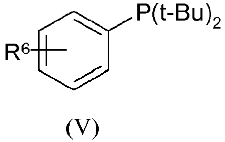 wobei
- R⁶: ausgewählt ist aus Wasserstoff, C₁-C₄ Alkyl, C₁-C₄ Haloalkyl, Phenyl und NR⁷ und
- R⁷: ausgewählt ist aus (C₁-C₄-Alkyl)₂
oder ein Salz hiervon, in der Suzuki-Reaktion nach Schema 1 mit hoher Selektivität und geringem Ausmaß an Triarylbildung chlorierte Biphenylanilide bzw. Biphenylaniline liefert.

### Organobor Verbindungen

Die Organoborverbindungen, die bei dem Verfahren gemäß der vorliegenden Erfindung verwendet werden können:
(i) Boronsäuren der Formel (III) in welchen
   - m: 2 ist,
   - p: 1 ist,
   - Q¹ und Q²: Hydroxylgruppen sind,
   - R² und n: wie oben definiert sind,
   oder die aus den Boronsäuren der Formel (III) gebildeten Anhydride, Dimere oder Trimere,
   sind durch Umwandlung von Arylmagnesiumhalogeniden mit Trialkylboraten, vorzugsweise in THF als Lösungsmittel, erhältlich. Zur Unterdrückung der Bildung von Arylborinsäuren ist es notwendig, keinen Überschuss der beiden Reagentien zu verwenden und die Umsetzung bei niedrigen Temperaturen von -60°C durchzuführen, wie in R.M. Washburn et al. Organic Syntheses Collective Band 4, 68, oder in Boronic Acids, Herausgeber Dennis G. Hall, Wiley-VCH 2005, S.28ff, und darin angegebenen Literaturstellen beschrieben.
   Als Beispiele für Boronsäuren, die gemäß der vorliegenden Erfindung verwendet werden können, seien die folgenden Verbindungen genannt:
   4-Chlorphenylboronsäure, 3-Chlorphenylboronsäure, 2-Chlorphenylboronsäure, 3,4-Dichlorphenylboronsäure und 2,3-Dichlorphenylboronsäure, insbesondere 3,4-Dichlorphenylboronsäure.
(ii) Boronsäurederivate der Formel (III), in welchen
   - m: 2 ist,
   - p: 1 ist,
   Q¹ und Q² jeweils unabhängig voneinander ausgewählt sind aus F, Cl, Br, I, C₁₋₄-Alkyl-, C₆₋₁₀-Aryl-, C₁₋₄-Alkoxy- und C₆₋₁₀-Aryloxy sind,
   R² und n wie oben definiert sind;
(iii) Borinsäuren der Formel (III), in welchen
   - m: 1 ist,
   - p: 2 ist,
   - Q: ausgewählt ist aus OH, F, Cl, Br, I, C₁₋₄-Alkyl-, C₁₋₄-Alkoxy- und C₆₋₁₀-Aryloxy-Resten,
   - R² und n: wie oben definiert sind,
   werden durch Umsetzung von gegebenenfalls substituiertem Phenylmagnesiumchlorid V mit Trialkylborat, vorzugsweise Trimethylborat, in Tetrahydrofuran als Lösungsmittel gemäß WO 2007/138089 erhalten, wie in Schema 3 beschrieben. 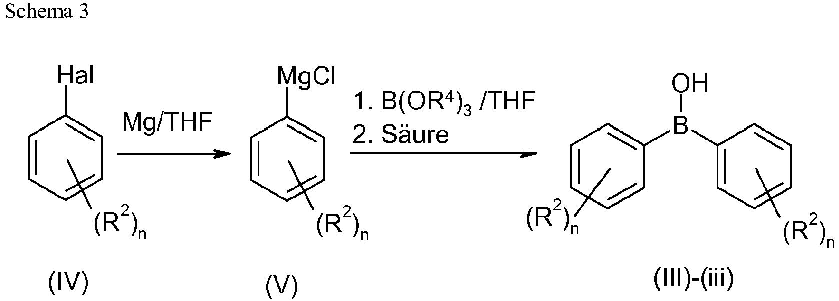
   - R⁴: steht für C₁-C₄-Alkyl, vorzugsweise Methyl.
   - Hal: steht für Cl, Br, I.

   Vorzugsweise geht man von Diphenylborinsäuren der Formel (iii) aus, worin m für 1 steht, p für 2 steht, Q für OH steht und R² und n die oben angegebenen Bedeutungen haben.
   Weitere Ausgangsstoffe sind Diphenylborinsäuren (iii), worin n für 1 oder 2, insbesondere 2, steht. Besonders bevorzugt sind Diphenylborinsäuren (iii), die in der 3- und 4-Position oder nur in der 4-Position substituiert sind.
   Borinsäuren, die gemäß der vorliegenden Erfindung verwendet werden können, sind ausgewählt aus der Gruppe bestehend aus Bis(3,4-Dichlorphenyl)borinsäure, Bis(2,3-Dichlorphenyl)borinsäure, Bis(3-Dichlorphenyl)borinsäure, Bis(4-Dichlorphenyl)borinsäure, 4-Chlorphenylboronsäure, 3-Chlorphenylboronsäure, 2-Chlorphenylboronsäure, 3,4-Dichlorphenylboronsäure und 2,3-Dichlorphenylboronsäure. Gemäß einer Ausführungsform der vorliegenden Erfindung ist die Verbindung der Formel (III) ausgewählt aus Bis(3,4-Dichlorphenyl)borinsäure und 4-Chlorphenylboronsäure.
   Wesentlich für eine hohe Ausbeute von Diphenylborinsäure (iii) ist der Einsatz von nur 0,7 Äq. Trialkylborat, bezogen auf das eingesetzte substituierte Chlorbenzol (IV). Bei einer Einsatzmenge von 1,1 Äq. Trialkylborat entsteht Phenylboronsäure, wie in der EP-A 0 888 261 beschrieben,
   Die Reaktionstemperatur bei dieser Verfahrensstufe liegt beispielsweise im Bereich von -20 bis 100°C, 20 bis 80°C oder 40 bis 60°C.
(iv) cyclische Boronsäureester der Formel (III), in welchen
   - m: 2 ist,
   - p: 1 ist,
   - Q¹ und Q²: jeweils unabhängig voneinander ausgewählt sind aus C₁₋₄-alkoxy Resten, die zusammen mit dem Boratom, an welches sie gebunden sind, einen 5- oder 6-gliedrigen Ring bilden, der mit C₁₋₄-Alkyl-Resten substituiert sein kann,
   - R² und n: wie oben definiert sind,
   sind gemäß Boronic Acids, Herausgeber Dennis G. Hall, Wiley-VCH 2005, S. 28ff und darin angegebenen Literaturstellen erhältlich.
   Als Beispiele für cyclische Boronsäureester, die gemäß der vorliegenden Erfindung verwendet werden können, seien Verbindungen gemäß den folgenden Formeln (iv-1) bis (iv-3) genannt: 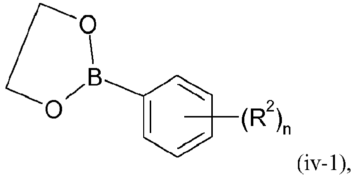 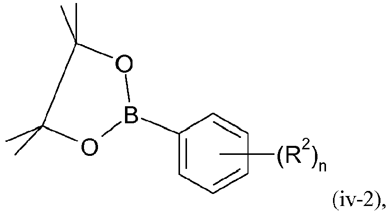 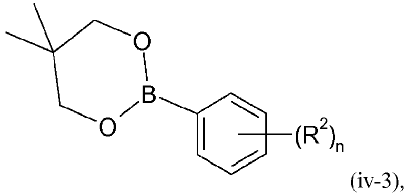 worin R² und n die oben angegebenen Bedeutungen haben.
(v) Boronate der Formel (III), in welchen
   - m: 3 ist,
   - p: 1 ist,
   - R² und n: wie oben definiert sind,
   - Q¹ bis Q³: jeweils unabhängig voneinander ausgewählt sind aus OH, F, Cl, Br, I, C₁₋₄-Alkyl-, C₁₋₄-Alkoxy- und C₆₋₁₀-Aryloxy-Resten,
   und worin die negative Ladung des Boronat Anions durch ein Kation kompensiert ist, wie durch die folgende Formel (iv-1) gezeigt.
   Das Kation (M⁺) ist beispielsweise aus der Gruppe bestehend aus Ammonium- (NH₄⁺), Alkali- oder Erdalkalimetallkationen, wie Na⁺, K⁺, Li⁺, Mg²⁺, Ca²⁺ ausgewählt.
   Die Boronate (v) sind gemäß Serwatowski et al., Tetrahedron Lett. 44, 7329 (2003), erhältlich.
(vi) Triarylborane der Formel (III), in welchen
   - m: 0 ist,
   - p: 3 ist,
   - R² und n: wie oben definiert sind.

   Die Triarylborane (vi) sind gemäß H.C. Brown et al., J. Organomet. Chem. 73, 1 (1988) und H.C. Brown et al., "Borane reagents", Verlag Harcourt Brace Jovanovich, (1988) erhältlich.
(vii) Tetraarylborate der Formel (III), in welchen
   - m: 0 ist,
   - p: 4 ist,
   - R² und n: wie oben definiert sind,
   und in welchen die negative Ladung des Boronat Anions durch ein Kation kompensiert ist welches beispielsweise aus der Gruppe bestehend aus Ammonium- (NH₄⁺), Alkali- oder Erdalkalimetall-Kationen, wie Na⁺, K⁺, Li⁺, Mg²⁺, Ca²⁺, ausgewählt ist.
   Die Tetraarylborate (vii) sind gemäß J. Serwatowski et al., Tetrahedron Lett. 44, 7329 (2003), erhältlich.

### Suzuki Kupplung

Gemäß der vorliegenden Erfindung können chlorierte Biphenylanilide und Biphenylaniline der Formel (I) in hoher Selektivität und mit hohen Ausbeuten hergestellt werden.

Durch Einsatz von Phosphinliganden der allgemeinen Formel (V) 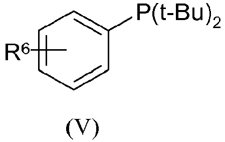 wobei
- R⁶: ausgewählt ist aus Wasserstoff, C₁-C₄ Alkyl, C₁-C₄ Haloalkyl, Phenyl und NR⁷ und
- R⁷: ausgewählt ist aus (C₁-C₄-Alkyl)₂
oder ein Salz hiervon,
ist es im Vergleich zur Verwendung aliphatischer Phosphinliganden möglich, den Anteil an Triarylen zu vermindern. R¹ kann dabei in Formel (I) bzw. (II) eine geschützte Aminogruppe, NO₂, NH₂ und NHR³ sein, wobei R³ wie oben definiert ist.

Wie in WO 2009/135598 offenbart kann die Suzuki-Kupplung bei Schützung der Aminogruppe des Arylhalogenids der Formel (II) durch eine Schutzgruppe unter milderen Reaktionsbedingungen durchgeführt werden. Daher wird die Bildung von unerwünschten Nebenprodukten, wie Dehalogenierungsprodukten, Triarylen und polychlorierten Biphenylen (PCB), erheblich verringert.

Schutzgruppe bedeutet in diesem Zusammenhang jede Art von chemischer Gruppe, die zur Modifizierung der Aminogruppe des Arylhalogenids der Formel (II) während des Schritts der Suzuki-Kupplung verwendet und nach der Kupplung unter Rückbildung des ursprünglichen Amins von dem substituierten Biphenylanilid der Formel (I) abgespalten werden kann, beispielsweise durch Umsetzung mit wässriger Säure. Dieser Schritt wird als Entschützung bezeichnet.

Als Beispiele für Schutzgruppen, die im Allgemeinen für den Schutz von Amingruppen eingesetzt werden können, seien die folgenden Gruppen genannt:
Schiff-Basen (RR"C=N-R'), die durch Reaktion der Aminogruppe mit einem Aldehyd oder Keton erhalten werden. Die Abspaltung der Schiff-Base-Schutzgruppe kann beispielsweise durch Säurebehandlung, durch Hydrierung mit Pd/C/Wasserstoff gemäß J. Am. Chem. Soc. 1960, 82, 5688, oder mit Hydrazin in Ethanol gemäß J. Chem. Soc. C, 1969, 1758, erfolgen.

Bevorzugt verwendet man Ketone wie Aceton, Benzophenon oder Pinakolon oder Aldehyde wie Formaldehyd, Acetaldehyd oder Benzaldehyd.

Acetylamino- und Acetacetylaminogruppen werden durch Reaktion der Aminogruppe mit Essigsäure oder mit Acetessigsäureestern erhalten. Die Abspaltung der Gruppen kann durch Säurebehandlung erfolgen.

In einer Ausführungsform der vorliegenden Erfindung ist die Aminogruppe des Arylhalogenids der Formel (II) durch eine Schiff-Base, durch eine Acetamino- oder durch eine Acetacetylaminogruppe geschützt.

In einer weiteren bevorzugten Ausführungsform der Erfindung
ist X ausgewählt aus Wasserstoff, Fluor und Chlor;
ist R¹ ausgewählt aus -NH(CO)R³, -N=CR⁴R⁵ und NH₂;
steht R² für Chlor;
sind R³, R⁴, R⁵ unabhängig voneinander ausgewählt aus Wasserstoff, -CH₂-(C=O)-C₁₋₈-Alkyl, C₁₋₈-Alkyl, C₁₋₈-Alkenyl, C₁₋₈-Alkinyl und C₆₋₁₈-Aryl, oder
können R⁴, R⁵ zusammen mit dem Kohlenstoffatom, an das sie gebunden sind, einen fünf- oder sechsgliedrigen Ring mit einem, zwei oder drei aus N, O oder S ausgewählten Heteroatomen bilden; und
ist n ausgewählt aus 1, 2 und 3.

In einer anderen Ausführungsform der Erfindung haben die nach dem erfindungsgemäßen Verfahren hergestellten substituierten Biphenyle die folgenden Substituenten, jeweils sowohl einzeln als auch in Kombination:
- X: ist ausgewählt aus Wasserstoff, Fluor und Chlor;
- R¹: ist ausgewählt aus -NH(CO)CH₃ und NH₂;
- R²: steht für Chlor;
- n: ist ausgewählt aus 1 und 2, vorzugsweise 2.

Die Durchführung der sich anschließenden homogen katalysierten Suzuku-Biaryl-Kreuzkupplung erfolgt gemäß Schema 1. 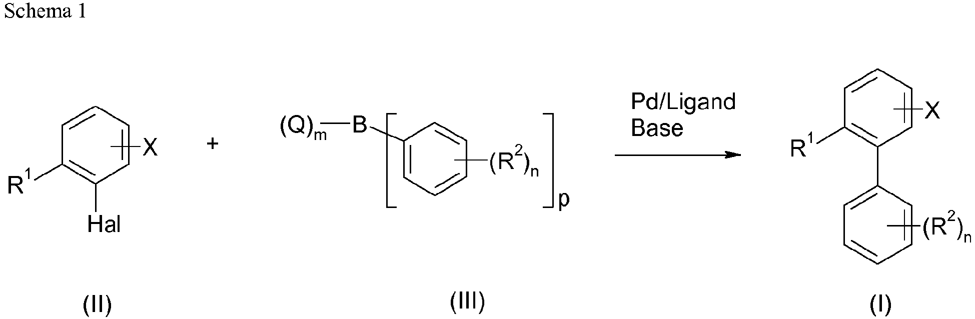 wobei als Ligand eine Verbindung der Formel (V) oder ein Salz hiervon eingesetzt wird. Die Substituenten sind wie oben beschrieben.

Der Phosphinligand der Formel (V) kann auch als Phosphonium-Salz wie z.B. als Tetrafluoroborat (Org. Lett. 2001, 3, 4295), Perchlorat oder Hydrogensulfat eingesetzt und hieraus *in situ* durch Base freigesetzt werden.

Die eingesetzte Base kann neben der Neutralisation der entstehenden Säure auch durch eine Aktivierung der Arylboronsäure zu anionischen Boronatspezies den Reaktionsverlauf positiv beeinflussen. Neben den oben genannten Basen kann eine solche Aktivierung auch durch Zusatz von Fluoridsalzen wie beispielsweise CaF, NaF, KF, LiF oder CsF erreicht werden.

Die eingesetzten Palladiumkatalysatoren werden in der Regel *in situ* aus mindestens einem Palladium(II)salz oder einer Palladium(0)-Verbindung und den entsprechenden Phosphin-Liganden erzeugt. Sie können jedoch auch als Palladium(0)-Verbindung direkt eingesetzt werden, ohne dass dadurch die anfängliche katalytische Aktivität gemindert wird.

Die unten stehenden Herstellungsbeispiele zeigen deutlich den überraschenden Vorteil des Aryl-Alkyl-Phosphinliganden der Formel (V), beispielsweise [(t-Bu)₂PhPH]BF₄, gegenüber einem Alkyl-Phosphinliganden, beispielsweise [(t-Bu)₃PH]BF₄. Der erfindungsgemäße Einsatz des Liganden der Formel (V) oder eines Salzes hiervon führt zu einer deutlich verringerten Bildung von Triarylen im Vergleich zum Einsatz von Alkyl-Phosphinliganden, beispielsweise [(t-Bu)₃PH]BF₄.

Beispiele der Verbindung (II) sind ausgewählt aus der Gruppe bestehend aus N-(2-Brom-4-fluorphenyl)acetamid, N-(2-Bromophenyl)acetamid, N-(2-bromphenyl)-3-oxobutanamid, N-(2-Bromo-4-fluorphenyl)-3-oxobutanamid, 2-Brom-N-(Propan-2-yliden)aniline, 2-Brom-4-Fluor-N-(Propan-2-yliden)anilin, N-(2-Brom-4-fluor)anilin, N-(2-bromo)anilin.

Gegenstand der vorliegenden Erfindung ist daher auch das oben beschriebene Verfahren, wobei die Verbindung (II) ausgewählt ist aus der Gruppe bestehend aus N-(2-Brom-4-fluorphenyl)acetamid, N-(2-Bromophenyl)acetamid, N-(2-bromphenyl)-3-oxobutanamid, N-(2-Bromo-4-fluorphenyl)-3-oxobutanamid, 2-Brom-N-(Propan-2-yliden)aniline, 2-Brom-4-Fluor-N-(Propan-2-yliden)anilin, N-(2-Brom-4-fluor)anilin, N-(2-bromo)anilin.

Die Verbindung (II) wird, bezogen auf die Organoborverbindung (III) (Boräquivalente), normalerweise äquimolar, vorzugsweise mit bis zu 20 prozentigem Überschuss, insbesondere mit bis zu 50 prozentigem Überschuss, ganz speziell mit bis zu 100 prozentigem Überschuss, verwendet.

Bevorzugte Verbindungen der Formel (III) umfassen sowohl Borinsäuren der Formel (III) (iii) als auch Boronsäuren der Formel (III) (i) oder (ii). Aus Gründen der Wirtschaftlichkeit ist der Einsatz von Borinsäuren der Formel (III) (iii) bevorzugt.

Beispiele für bevorzugte Verbindungen der Formel (III) sind ausgewählt aus der Gruppe bestehend aus Bis(3,4-Dichlorphenyl)borinsäure, Bis(2,3-Dichlorphenyl)borinsäure, Bis(3-Dichlorphenyl)borinsäure, Bis(4-Dichlorphenyl)borinsäure, 4-Chlorphenylboronsäure, 3-Chlorphenylboronsäure, 2-Chlorphenylboronsäure, 3,4-Dichlorphenylboronsäure und 2,3-Dichlorphenylboronsäure.

Bevorzugte Verbindungen der Formel (III) sind auch ausgewählt aus der Gruppe bestehend aus Bis(3,4-Dichlorphenyl)borinsäure, Bis(2,3-Dichlorphenyl)borinsäure, Bis(3-Dichlorphenyl)borinsäure, und Bis(4-Dichlorphenyl)borinsäure.

Beispiele für Kombinationen von Verbindungen (II) und (III) gemäß der vorliegenden Erfindung sind:
Verbindung (II) ist 2-Brom-4-fluoracetanilid und Verbindung (III) ist ausgewählt aus der Gruppe bestehend aus Bis(3,4-dichlorphenyl)borinsäure, Bis(2,3-Dichlorphenyl)borinsäure, Bis(3-Dichlorphenyl)borinsäure, Bis(4-Dichlorphenyl)borinsäure, 4-Chlorphenylboronsäure, 3-Chlorphenylboronsäure, 2-Chlorphenylboronsäure, 3,4-Dichlorphenylboronsäure und 2,3-Dichlorphenylboronsäure.

Verbindung (II) ist 2-Brom-4-fluoranilin und Verbindung (III) ist ausgewählt aus der Gruppe bestehend aus Bis(3,4-dichlorphenyl)borinsäure, Bis(2,3-Dichlorphenyl)borinsäure, Bis(3-Dichlorphenyl)borinsäure, Bis(4-Dichlorphenyl)borinsäure, 4-Chlorphenylboronsäure, 3-Chlorphenylboronsäure, 2-Chlorphenylboronsäure, 3,4-Dichlorphenylboronsäure und 2,3-Dichlorphenylboronsäure.

Verbindung (II) ist 2-Brom-acetanilid und Verbindung (III) ist ausgewählt aus der Gruppe bestehend aus Bis(3,4-dichlorphenyl)borinsäure, Bis(2,3-Dichlorphenyl)borinsäure, Bis(3-Dichlorphenyl)borinsäure, Bis(4-Dichlorphenyl)borinsäure, 4-Chlorphenylboronsäure, 3-Chlorphenylboronsäure, 2-Chlorphenylboronsäure, 3,4-Dichlorphenylboronsäure und 2,3-Dichlorphenylboronsäure.

Verbindung (II) ist 2-Bromanilin und Verbindung (III) ist ausgewählt aus der Gruppe bestehend aus Bis(3,4-dichlorphenyl)borinsäure, Bis(2,3-Dichlorphenyl)borinsäure, Bis(3-Dichlorphenyl)borinsäure, Bis(4-Dichlorphenyl)borinsäure, 4-Chlorphenylboronsäure, 3-Chlorphenylboronsäure, 2-Chlorphenylboronsäure, 3,4-Dichlorphenylboronsäure und 2,3-Dichlorphenylboronsäure.

Gemäß einer weiteren Ausführungsform der vorliegenden Erfindung ist die Verbindung (II) 2-Brom-4-fluoracetanilid und die Verbindung (III) ist Bis(3,4-dichlorphenyl)borinsäure.

Gemäß einer weiteren Ausführungsform der vorliegenden Erfindung ist die Verbindung (II) 2-Brom-4-fluoranilin und die Verbindung (III) ist Bis(3,4-dichlorphenyl)borinsäure.

Gemäß einer weiteren Ausführungsform der vorliegenden Erfindung ist die Verbindung (II) 2-Brom-acetanilid und die Verbindung (III) ist 4-Chlorphenylboronsäure

Gemäß einer weiteren Ausführungsform der vorliegenden Erfindung ist die Verbindung (II) 2-Bromanilin und die Verbindung (III) ist 4-Chlorphenylboronsäure.

Als Basen können organische Basen, beispielsweise tertiäre Amine, eingesetzt werden. Bevorzugt verwendet man beispielsweise Triethylamin oder Dimethylcyclohexylamin. Als Basen verwendet man vorzugsweise Alkalimetallhydroxide, Erdalkalimetallhydroxide, Alkalimetallcarbonate, Erdalkalimetallcarbonate, Alkalimetallhydrogencarbonate, Alkalimetallacetate, Erdalkalimetallacetate, Alkalimetallalkoholate und Erdalkalimetallalkoholate, im Gemisch und insbesondere einzeln. Als Basen besonders bevorzugt sind Alkalimetallhydroxide, Erdalkalimetallhydroxide, Alkalimetallcarbonat, Erdalkalimetallcarbonat und Alkalimetallhydrogencarbonate. Als Basen insbesondere bevorzugt sind Alkalimetallhydroxide, z.B. Natriumhydroxid und Kaliumhydroxid, sowie Alkalimetallcarbonate und Alkalimetallhydrogencarbonate, z.B. Lithiumcarbonat, Natriumcarbonat und Kaliumcarbonat. Demnach ist gemäß einer Ausführungsform der vorliegenden Erfindung die Base ausgewählt aus Alkalimetall Hydroxiden, Alkalimetall Carbonaten und Alkalimetall Hydrogencarbonaten. Gemäß einer weiteren Ausführungsform der vorliegenden Erfindung ist die Base ausgewählt aus NaOH, KOH, Li₂CO₃, Na₂CO₃ und K₂CO₃. Die Base wird bei dem erfindungsgemäßen Verfahren vorzugsweise mit einem Anteil von 100 bis 500 mol-%, weiter bevorzugt 150 bis 400 mol-%, bezogen auf die Menge von Organoborverbindung (III), eingesetzt.

Geeignete Palladiumkatalysatoren sind Palladium-Ligand-Komplexe mit Palladium in der Oxidationsstufe Null, Palladiumsalze in Gegenwart von Komplexliganden oder gegebenenfalls auf Träger aufgezogenes metallisches Palladium, in Anwesenheit von Phosphinliganden der allgemeinen Formel (V) 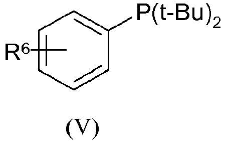 wobei
- R⁶: ausgewählt ist aus Wasserstoff, C₁-C₄Alkyl, C₁-C₄Haloalkyl, Phenyl und NR⁷ und
- R⁷: ausgewählt ist aus (C₁-C₄-Alkyl)₂,
oder ein Salz hiervon.

Gemäß einer weiteren Ausführungsform der vorliegenden Erfindung ist
- R⁶: ausgewählt aus Wasserstoff, Methyl, Difluormethyl und Trifluormethyl, und
- R⁷: ist (CH₃)₂.

Gemäß einer Ausführungsform der vorliegenden Erfindung sind die Phosphinliganden der allgemeinen Formel (V) ausgewählt aus Di(tert.-butyl)phenylphosphin, Di-tert-butyl-p-[4-(trifluoromethyl)phenyl]phosphin, 4-(Di-tert-butylphosphino)-p-N,N-dimethylanilin und Di-tert-butyl-p-(4-methylphenyl)phosphin.

Gemäß einer weiteren Ausführungsform der vorliegenden Erfindung ist der Phosphinligand der allgemeinen Formel (V) Di(tert.-butyl)phenylphosphin.

Im Rahmen der vorliegenden Erfindung wird auch eine Verbindung der Formel (V) offenbart 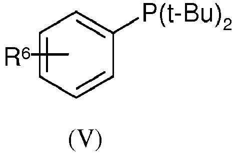 wobei
- R⁶: ausgewählt ist aus Wasserstoff, C₁-C₄Alkyl, C₁-C₄Haloalkyl, Phenyl und NR⁷ und
- R⁷: ausgewählt ist aus (C₁-C₄-Alkyl)₂.

Weiterhin wird im Rahmen der vorliegenden Erfindung eine Verbindung der Formel (V) offenbart 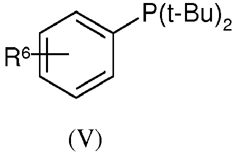 wobei
- R⁶: ausgewählt ist aus Wasserstoff, Methyl, 1- bis 3-fach halogeniertem Methyl und NR⁷ und
- R⁷: (CH₃)₂ ist.

Die in Formel (V) dargestellten Phosphine können auch in Form ihrer Salze, wie z. B. als Tetrafluoroborat, Perchlorat oder Hydrogensulfat eingesetzt werden und durch Zusatz einer Base *in situ* freigesetzt werden.

Weiterhin wird im Rahmen der vorliegenden Erfindung eine Verbindung der Formel (V-i) offenbart 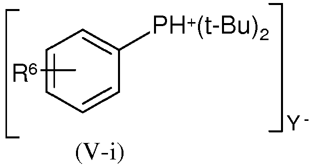 wobei
- R⁶: ausgewählt ist aus Wasserstoff, C₁-C₄Alkyl, C₁-C₄Haloalkyl, Phenyl und NR⁷ und
- R⁷: ausgewählt ist aus (C₁-C₄-Alkyl)₂ und
- Y: ausgewählt ist aus der Gruppe bestehend aus BF₄⁻, Perchlorat und Hydrogensulfat.

Gemäß einer Ausführungsform der vorliegenden Erfindung ist der Palladium Katalysator ausgewählt aus der Gruppe bestehend aus:
a) einem Komplex bestehend aus Palladium in der Oxidationsstufe 0 und einem Phosphinliganden der Formel (V) oder einem Salz hiervon,
b) einem Palladiumsalz in Gegenwart eines Phosphinliganden der Formel (V) oder einem Salz hiervon und
c) metallischem, gegebenenfalls auf einen Träger aufgebrachtem Palladium, in Gegenwart eines Phosphinliganden der Formel (V) oder einem Salz hiervon.

Eine Ausführungsform der vorliegenden Erfindung ist das oben beschriebene Verfahren, wobei der Palladium Katalysator a) ein Komplex aus Palladium im Oxidationszustand 0 und einem Phosphinliganden der Formel (V) oder ein Salz hiervon ist.

Eine weitere Ausführungsform der vorliegenden Erfindung ist das oben beschriebene Verfahren, wobei ein Palladium Katalysator b) verwendet wird. Gemäß einer weiteren Ausführungsform ist das Salz des Palladium Katalysators b) ausgewählt aus der Gruppe bestehend aus Palladiumchlorid, Palladiumacetat, Palladiumacetylacetonat und Bisacetonitril-Palladiumchlorid.

Eine weitere Ausführungsform der vorliegenden Erfindung ist das oben beschriebene Verfahren, wobei ein Palladium Katalysator c) verwendet wird und dieser Palladium Katalysator c) aus metallischem Palladium auf aktiviertem Kohlenstoff in Anwesenheit eines Phosphinliganden der allgemeinen Formel (V) oder eines Salzes hiervon besteht.

Die Reaktivität der Komplexliganden kann durch Zusatz eines quartären Ammoniumsalzes wie Tetra-n-butylammoniumbromid (TBAB) gesteigert werden (siehe beispielsweise D. Zim et al., Tetrahedron Lett. 2000, 41, 8199). Bei Bedarf kann die Wasserlöslichkeit der Palladiumkomplexe durch verschiedene Substituenten verbessert werden, wie Sulfonsäure- oder Sulfonsäuresalzgruppen, Carbonsäure- oder Carbonsäuresalzgruppen, Phosphonsäure-, Phosphonium- oder Phosphonsäuresalzgruppen, Peralkylammonium-, Hydroxy- und Polyethergruppen.

Aus den Palladium-Ligand-Komplexen mit Palladium in der Oxidationsstufe Null verwendet man vorzugsweise Tetrakis(triphenylphosphin)palladium und daneben Tetrakis[tri(o-tolyl)phosphin]palladium. In den Palladiumsalzen, die in Gegenwart von Komplexliganden verwendet werden, liegt das Palladium normalerweise in der zweifach positiven Oxidationsstufe vor. Gemäß einer bevorzugten Ausführungsform der vorliegenden Erfindung ist der Palladium Katalysator b) ausgewählt aus der Gruppe bestehend aus palladium chloride, palladium acetate, Palladiumacetylacetonat or bisacetonitrilepalladium chloride. Particular preference is given to using Palladiumacetylacetonat.

Das molare Verhältnis von Palladium zum Phosphin-Liganden der Formel (V) oder eines seiner Salze sollte zwischen 4:1 und 1:100 liegen, und liegt vorzugsweise zwischen 1 : 1 und 1 : 5, besonders bevorzugt zwischen 1 : 1 und 1 : 2.

Besonders bevorzugt ist bei der Verwendung von gegebenenfalls auf Träger aufgezogenem metallischem Palladium die Mitverwendung der vorstehend genannten Phosphinliganden der Formel (V) oder (V-i). Der Palladiumkatalysator wird bei dem erfindungsgemäßen Verfahren mit einem niedrigen Anteil von 0,001 bis 1,0 mol-%, vorzugsweise 0,005 bis 0,5 mol-% oder 0,01 bis 0,5 mol-% und insbesondere 0,005 bis 0,05 mol-%, bezogen auf die Menge von Verbindung (II), eingesetzt.

Das erfindungsgemäße Verfahren kann in einem Zweiphasensystem aus wässriger Phase und fester Phase, d.h. dem Katalysator, durchgeführt werden. Die wässrige Phase kann dabei neben Wasser auch ein wasserlösliches organisches Lösungsmittel enthalten.

Für das erfindungsgemäße Verfahren geeignete organische Lösungsmittel sind Ether, wie Dimethoxyethan, Diethylenglykoldimethylether, Tetrahydrofuran, Dioxan und tert.-Butylmethylether, Kohlenwasserstoffe wie n-Hexan, n-Heptan, Cyclohexan, Benzol, Toluol und Xylol, Alkohole wie Methanol, Ethanol, 1-Propanol, 2-Propanol, Ethylenglykol, 1-Butanol, 2-Butanol und tert.-Butanol, Ketone wie Aceton, Ethylmethylketon und Isobutylmethylketon, Amide wie Dimethylformamid, Dimethylacetamid und N-Methylpyrrolidon, jeweils einzeln oder im Gemisch.

Bevorzugte Lösungsmittel sind Ether, wie Dimethoxyethan, Tetrahydrofuran und Dioxan, Kohlenwasserstoffe wie Cyclohexan, Toluol und Xylol, Alkohole wie Ethanol, 1- Propanol, 2-Propanol, 1-Butanol und tert.-Butanol, jeweils einzeln oder im Gemisch. In einer besonders bevorzugten Variante des erfindungsgemäßen Verfahrens verwendet man ein oder mehrere wasserunlösliche und ein oder mehrere wasserlösliche Lösungsmittel, beispielsweise Gemische aus Wasser und Dioxan oder Wasser und Tetrahydrofuran oder Wasser, Dioxan und Ethanol oder Wasser, Tetrahydrofuran und Methanol oder Wasser, Toluol und Tetrahydrofuran, vorzugsweise Wasser und Tetrahydrofuran oder Wasser, Tetrahydrofuran und Methanol.

Gemäß einer Ausführungsform der vorliegenden Erfindung wird die Reaktion in einer Mischung aus Wasser und einem organischen Lösungsmittel durchgeführt. Gemäß einer weiteren Ausführungsform der vorliegenden Erfindung wird die Reaktion in einer Mischung aus Wasser und 1-Butanol durchgeführt.

Die Gesamtmenge an Lösungsmittel liegt normalerweise bei 3000 bis 500 g und vorzugsweise 2000 bis 700 g pro Mol der Verbindung (II).

Zweckmäßigerweise werden zur Durchführung des Verfahrens die Verbindung (II), die Organoborverbindung (III), die Base und die katalytisch wirksame Menge des Palladiumkatalysators in ein Gemisch aus Wasser und einem oder mehreren inerten organischen Lösungsmitteln gegeben und bei einer Temperatur von 20°C bis 100°C, vorzugsweise 50°C bis 90°C, weiter bevorzugt 60°C bis 80°C, über einen Zeitraum von 1 bis 50 Stunden, vorzugsweise 2 bis 24 Stunden, gerührt.

Je nach verwendetem Lösungsmittel und verwendeter Temperatur stellt sich ein Druck von 1 bar bis 6 bar, vorzugsweise 1 bar bis 4 bar, ein. Bevorzugt wird die Umsetzung in Wasser und Tetrahydrofuran durchgeführt. Die Umsetzung kann in üblichen Apparaturen, die für derartige Verfahren geeignet sind, durchgeführt werden. Nach beendeter Umsetzung wird als Feststoff anfallender Palladiumkatalysator beispielsweise durch Filtration abgetrennt und das Rohprodukt vom Lösungsmittel bzw. den Lösungsmitteln befreit. Bei nicht völlig wasserlöslichen Produkten werden wasserlösliche Palladiumkatalysatoren oder Komplexliganden bei der Trennung der Wasserphase vom Rohprodukt vollständig abgetrennt. Anschließend kann nach dem Fachmann bekannten und dem jeweiligen Produkt angemessenen Methoden eine weitere Aufreinigung erfolgen, beispielsweise durch Umkristallisation, Destillation, Sublimation, Zonenschmelzen, Schmelzekristallisation oder Chromatographie.

Mit dem erfindungsgemäßen Verfahren können beispielsweise die folgenden Verbindungen hergestellt werden:
N-(3',4'-Dichlor-5-fluorbiphenyl-2-yl)acetamid, 3',4'-Dichlor-5-fluorbiphenyl-2-amin, N-(4'-Chlorbiphenyl-2-yl)acetamid, 4'-Chlorbiphenyl-2-amin.

Weitere Beispiele sind: 3',4'-Dichlor-5-fluor-N-(propan-2-yliden)biphenyl-2-amin, 3',4'-Dichlor-N-(propan-2-yliden)biphenyl-2-amin, 4'-Chlor-N-(propan-2-yliden)biphenyl-2-amin, N-(4'-Chlor-5-fluorbiphenyl-2-yl)acetamid, N-(3',4'-Dichlor-biphenyl-2-yl)acetamid.

Das erfindungsgemäße Verfahren liefert die Verbindungen der Formel (I) in sehr hohen quantitativen Ausbeuten bei sehr guter Reinheit. Die mit dem erfindungsgemäßen Verfahren erhältlichen substituierten Biphenyle eignen sich als Vorprodukte für fungizide Pflanzenschutz-Wirkstoffe (siehe WO 03/070705). Bei Verwendung einer Amin-Schutzgruppe wird diese in den meisten Fällen wird die Amin-Schutzgruppe vor der weiteren Umwandlung der Amine abgespalten.

### Herstellungsbeispiele

### Allgemeine Arbeitsvorschrift zur Biarylsynthese

Unter Argon wird eine Mischung aus Anilin bzw. Acetanilid (1,0 Äquivalent), chlorierte Diphenylborinsäure 0,5 Äquivalente) od. chlorierte Phenylboronsäure (1,0 Äquivalent), Base, [(t-Bu)3PH]BF4 bzw. [(t-Bu)2PhPH]BF4 (0,12 mol%), Pd(acac)2 (0,12 mol%) in 8 ml Wasser und 2 ml 1-Butanol auf 60°C erwärmt. Die Reaktionsmischung wird ca. 20 Stunden bei 60°C gerührt, auf Raumtemperatur abgekühlt und mit 1 N Salzsäure angesäuert. Nach zweimaliger Extraktion der Reaktionsmischung mit Ethylacetat werden die vereinigten organischen Phasen über Magnesiumsulfat getrocknet. Das Lösungsmittel wird im Vakuum abdestilliert.

Entsprechend der allgemeinen Arbeitsvorschrift wurden folgende Biaryle hergestellt:
1. *N*-(3',4'-dichloro-5-fluorobiphenyl-2-yl)acetamid (Biaryl 1)
2. *3',4'*-Dichlor-5-fluorbiphenyl-2-amin (Biaryl 2)
3. *N*-(4'-Chlorbiphenyl-2-yl)acetamid (Biaryl 3)
4. *4'*-Chlorbiphenyl-2-amin (Biaryl 4)

Aus Tabelle 1 gehen Ansatzgröße und Reagenzien hervor.

**Tabelle 1**

| **Biaryl** | **Anilin/Anilid** | **Ansatzgröße [mmol]** | **Borverbindung** | **Base** | **Äquivalente Base** |
|---|---|---|---|---|---|
| **1** | A-1 | 4,3 | B-1 | K₂CO₃ | 1,74 |
| **2** | A-2 | 5,7 | B-1 | K₂CO₃ | 1,74 |
| **3** | A-3 | 4,6 | B-2 | Phosphatpuffer pH 7-10 | 2,18 20ml/0,5M |
| **4** | A-4 | 5,7 | B-2 | K₂CO₃ | 1,74 |

### Anilin/Anilid

A-1: 2-Brom-4-fluoracetanilid
A-2: 2-Brom-4-fluoranilin
A-3: 2-Brom-acetanilid
A-4: 2-Bromanilin

### Borverbindung

B-1: Bis(3,4-dichlorphenyl)borinsäure
B-2: (4-Chlorphenyl)boronsäure

Tabelle 2 zeigt den Anteil (Fl.%, GC-MS) gebildeter Triaryle in der Reaktionsmischung bei Herstellung der Biaryle 1 bis 4 in Abhängigkeit des verwendeten Liganden.

**Tabelle 2**

| **Biaryl** | **1** | | **2** | | **3** | | **4** | |
|---|---|---|---|---|---|---|---|---|
| **Ligand** | A | B | A | B | A | B | A | B |
| **Triaryl [Fl.%, GC-MS]** | 5,6 | 3,3 | 2,1 | 0,9 | 0,3 | 0 | 3,5 | 0,5 |

| | | | | | | | | |
|---|---|---|---|---|---|---|---|---|
| Ligand A: [(t-Bu)₃PH]BF₄ Ligand B: [(t-Bu)₂PhPH]BF₄ | | | | | | | | |

### Beispiel

### Herstellung von N-(3',4'-dichloro-5-fluorobiphenyl-2-yl)acetamid

Unter Argon wurde eine Mischung aus *N*-(2-bromo-4-fluorophenyl)acetamid (1,00 g, 4,27 mmol), Bis(3,4-dichlorphenyl)borinsäure (0,685 g, 2,14 mmol), Kaliumcarbonat (1,03 g, 7,44 mmol), [(t-Bu)₂PhPH]BF₄ (1,6 mg, 5,2 µmol), Pd(acac)₂ (1,6 mg, 5,3 µmol) in 8 ml Wasser und 2 ml 1-Butanol auf 60°C erwärmt. Die Reaktionsmischung wurde ca. 13 Stunden bei 60°C gerührt, auf Raumtemperatur abgekühlt und mit 1 N Salzsäure angesäuert. Nach zweimaliger Extraktion der Reaktionsmischung mit Ethylacetat wurden die vereinigten organischen Phasen über Magnesiumsulfat getrocknet. Das Lösungsmittel wurde im Vakuum abdestilliert. Es wurden 1,21 g Rohprodukt erhalten (90,8 Fl.% HPLC, 86 % Ausbeute).

## Patentansprüche

1. Verfahren zur Herstellung halogenierter Biphenylanilide der Formel (I) 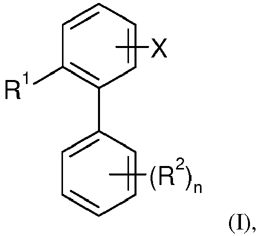 worin
X ausgewählt ist aus Wasserstoff, Fluor und Chlor;
R¹ ausgewählt ist aus -NH(CO)R³, -N=CR⁴R⁵, NO₂, NH₂ und NHR³;
R² Chlor ist;
R³, R⁴, R⁵ unabhängig voneinander ausgewählt sind aus Wasserstoff, -CH₂-(C=O)CH₃, C₁-C₈-Alkyl, C₁-C₈-Alkenyl, C₁-C₈-Alkynyl und C₆-C₁₈-Aryl; oder
R⁴, R⁵ zusammen mit dem Kohlenstoffatom, an das sie gebunden sind einen 5- oder 6-gliedrigen Ring bilden können, der 1, 2 oder 3 Heteroatome ausgewählt aus N, O, und S enthält;
n ausgewählt ist aus 1, 2 und 3,
durch Umsetzung einer Verbindung der Formel (II) 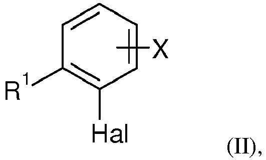 worin
Hal ausgewählt ist aus Brom und Jod; und R¹ und X wie oben definiert sind,
in Gegenwart einer Base und eines Palladium Katalysators ausgewählt aus der Gruppe bestehend aus:
a) einem Komplex bestehend aus Palladium in der Oxidationsstufe 0 und einem Phosphinliganden der Formel (V) oder einem Salz hiervon,
b) einem Palladiumsalz in Gegenwart eines Phosphinliganden der Formel (V) oder einem Salz hiervon und
c) metallischem, gegebenenfalls auf einen Träger aufgebrachtem Palladium, in Gegenwart eines Phosphinliganden der Formel (V) oder einem Salz hiervon,
wobei der Phosphinligand der Formel (V) wie folgt definiert ist 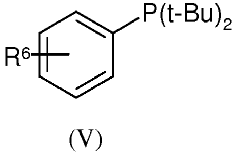 wobei
R⁶ ausgewählt ist aus Wasserstoff, C₁-C₄Alkyl, C₁-C₄Haloalkyl, Phenyl und NR⁷ und
R⁷ ausgewählt ist aus (C₁-C₄-Alkyl)₂,
oder ein Salz hiervon
in einem Lösungsmittel, mit einer Organoborverbindung der Formel (III) 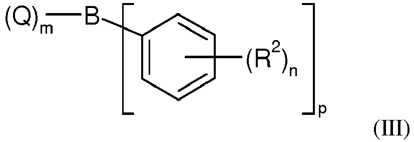 ausgewählt aus der Gruppe bestehend aus:
(i) Boronsäuren der Formel (III) in welchen
m 2 ist,
p 1 ist,
Q¹ und Q² Hydroxylgruppen sind,
R² und n wie oben definiert sind,
oder die aus den Boronsäuren der Formel (III) gebildeten Anhydride, Dimere oder Trimere;
(ii) Boronsäurederivate der Formel (III), in welchen
m 2 ist,
p 1 ist,
Q¹ und Q² jeweils unabhängig voneinander ausgewählt sind aus F, Cl, Br, I, C₁₋₄-Alkyl-, C₆₋₁₀-Aryl-, C₁₋₄-Alkoxy- und C₆₋₁₀-Aryloxy sind,
R² und n wie oben definiert sind;
(iii) Borinsäuren der Formel (III), in welchen
m 1 ist,
p 2 ist,
Q ausgewählt ist aus OH, F, Cl, Br, I, C₁₋₄-Alkyl-, C₆₋₁₀-Aryl-, C₁₋₄-Alkoxy- und C₆₋₁₀-Aryloxy-Resten,
R² und n wie oben definiert sind;
(iv) cyclische Boronsäureester der Formel (III), in welchen
m 2 ist,
p 1 ist,
Q¹ und Q² jeweils unabhängig voneinander ausgewählt sind aus C₁₋₄-Alkoxy Resten, die zusammen mit dem Boratom, an welches sie gebunden sind, einen 5- oder 6-gliedrigen Ring bilden, der mit C₁₋₄-Alkyl-Resten substituiert sein kann,
R² und n wie oben definiert sind;
(v) Boronate der Formel (III), in welchen
m 3 ist,
p 1 ist,
R² und n wie oben definiert sind,
Q¹ bis Q³ jeweils unabhängig voneinander ausgewählt sind aus OH, F, Cl, Br, I, C₁₋₄-Alkyl-, C₆₋₁₀-Aryl-, C₁₋₄-Alkoxy- und C₆-₁₀-Aryloxy-Resten,
und worin die negative Ladung des Boronat Anions durch ein Kation kompensiert ist;
(vi) Triarylborane der Formel (III), in welchen
m 0 ist,
p 3 ist,
R² und n wie oben definiert sind;
(vii) Tetraarylborate der Formel (III), in welchen
m 0 ist,
p 4 ist,
R² und n wie oben definiert sind,
und in welchen die negative Ladung des Boronat Anions durch ein Kation kompensiert ist.

2. Verfahren nach Anspruch 1, wobei die Verbindung (II) ausgewählt ist aus der Gruppe bestehend aus N-(2-Brom-4-fluorphenyl)acetamid, N-(2-Bromophenyl)acetamid, N-(2-bromphenyl)-3-oxobutanamid, N-(2-Bromo-4-fluorphenyl)-3-oxobutanamid, 2-Brom-N-(Propan-2-yliden)aniline, 2-Brom-4-Fluor-N-(Propan-2-yliden)anilin, N-(2-Brom-4-fluor)anilin, N-(2-bromo)anilin.

3. Verfahren nach Anspruch 1 oder 2, wobei die Verbindung der Formel (III) ausgewählt ist aus der Gruppe bestehend aus Bis(3,4-Dichlorphenyl)borinsäure, Bis(2,3-Dichlorphenyl)borinsäure, Bis(3-Dichlorphenyl)borinsäure, Bis(4-Dichlorphenyl)borinsäure, 4-Chlorphenylboronsäure, 3-Chlorphenylboronsäure, 2-Chlorphenylboronsäure, 3,4-Dichlorphenylboronsäure und 2,3-Dichlorphenylboronsäure.

4. Verfahren nach einem der Ansprüche 1 bis 3, wobei die Base ausgewählt ist aus Alkalimetall Hydroxiden, Alkalimetall Carbonaten und Alkalimetall Hydrogencarbonaten.

5. Verfahren nach einem der Ansprüche 1 bis 4, wobei der Palladium Katalysator a) gemäß Anspruch 1 ein Komplex aus Palladium im Oxidationszustand 0 und einem Phosphinliganden der Formel (V) oder einem Salz hiervon ist.

6. Verfahren nach einem der Ansprüche 1 bis 4, wobei ein Palladium Katalysator b) gemäß Anspruch 1 verwendet wird.

7. Verfahren nach einem der Ansprüche 1 bis 4, wobei ein Palladium Katalysator c) gemäß Anspruch 1 verwendet wird und dieser Palladium Katalysator c) aus metallischem Palladium auf aktiviertem Kohlenstoff in Anwesenheit eines Phosphinliganden der allgemeinen Formel (V) oder einem Salz hiervon besteht.

8. Verfahren nach Anspruch 6, wobei das Salz des Palladium Katalysators b) ausgewählt ist aus der Gruppe bestehend aus Palladiumchlorid, Palladiumacetate, Palladiumacetylacetonat und Bisacetonitril-Palladiumchloride.

9. Verfahren nach einem der Ansprüche 1 bis 8, wobei der Phosphinligand der allgemeinen Formel (V) ausgewählt ist aus Di(tert.-butyl)phenylphosphin, Di-tert-butyl p-[4-(trifluoromethyl)phenyl]phosphin, 4-(Di-tert-butylphosphino)-p-N,N-dimethylanilin und Di-tert-butyl-p-(4-methylphenyl)phosphin.

10. Verfahren nach einem der Ansprüche 1 bis 4, 6 oder 8, wobei ein Palladium Katalysator b) verwendet wird, wobei das molare Verhältnis des Palladium Salzes zu dem Phosphinliganden der allgemeinen Formel (V) oder einem Salz hiervon 1 : 1 bis 1 : 5 beträgt..

11. Verfahren nach einem der Ansprüche 1 bis 10, wobei 0.001 bis 1.0 mol% des Palladium Katalysators bezogen auf die Menge der Verbindung der Formel (II) verwendet werden.

12. Verfahren nach einem der Ansprüche 1 bis 11, wobei die Reaktion bei einer Temperatur von 20 bis 100°C durchgeführt wird.

13. Verfahren nach einem der Ansprüche 1 bis 12, wobei die Reaktion in einer Mischung aus Wasser und einem organischen Lösungsmittel durchgeführt wird.

## Claims

1. Process for preparing halogenated biphenylanilides of the formula (I) 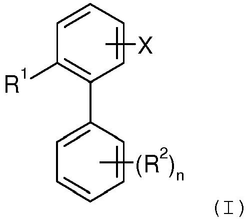 in which
X is selected from hydrogen, fluorine and chlorine;
R¹ is selected from -NH(CO)R³, -N=CR⁴R⁵, NO₂, NH₂ and NHR³;
R² is chlorine;
R³, R⁴, R⁵ are each independently selected from hydrogen, -CH₂-(C=O)CH₃, C₁-C₈-alkyl, C₁-C₈-alkenyl, C₁-C₈-alkynyl and C₆-C₁₈-aryl; or
R⁴, R⁵, together with the carbon atom to which they are bonded, may form a 5- or 6-membered ring containing 1, 2 or 3 heteroatoms selected from N, O and S;
n is selected from 1, 2 and 3,
by reacting a compound of the formula (II) 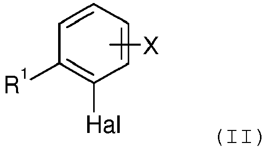 in which
Hal is selected from bromine and iodine; and R¹ and X are each as defined above,
in the presence of a base and a palladium catalyst selected from the group consisting of:
a) a complex consisting of palladium in the 0 oxidation state and a phosphine ligand of the formula (V) or a salt thereof,
b) a palladium salt in the presence of a phosphine ligand of the formula (V) or a salt thereof and
c) metallic palladium, optionally applied to a support, in the presence of a phosphine ligand of the formula (V) or a salt thereof,
where the phosphine ligand of the formula (V) is defined as follows: 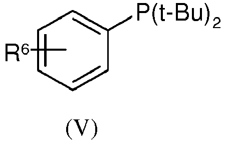 where
R⁶ is selected from hydrogen, C₁-C₄ alkyl, C₁-C₄ haloalkyl, phenyl and NR⁷ and
R⁷ is selected from (C₁-C₄-alkyl)₂,
or a salt thereof,
in a solvent, with an organoboron compound of the formula (III) 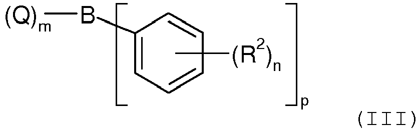
selected from the group consisting of:
(i) boronic acids of the formula (III) in which
m is 2,
p is 1,
Q¹ and Q² are each hydroxyl groups,
R² and n are each as defined above,
or the anhydrides, dimers or trimers formed
from the boronic acids of the formula (III);
(ii) boronic acid derivatives of the formula (III) in which
m is 2,
p is 1,
Q¹ and Q² are each independently selected from F, Cl, Br, I, C₁₋₄-alkyl-, C₆₋₁₀-aryl-, C₁₋₄-alkoxy- and C₆₋₁₀-aryloxy,
R² and n are each as defined above;
(iii) borinic acids of the formula (III) in which
m is 1,
p is 2,
Q is selected from OH, F, Cl, Br, I, C₁₋₄-alkyl, C₆₋₁₀-aryl, C₁₋₄-alkoxy and C₆₋₁₀-aryloxy radicals,
R² and n are each as defined above;
(iv) cyclic boronic esters of the formula (III) in which
m is 2,
p is 1,
Q¹ and Q² are each independently selected from C₁₋₄-alkoxy radicals which, together with the boron atom to which they are bonded, form a 5- or 6-membered ring which may be substituted by C₁₋₄-alkyl radicals,
R² and n are each as defined above;
(v) boronates of the formula (III) in which
m is 3,
p is 1,
R² and n are each as defined above,
Q¹ to Q³ are each independently selected from OH, F, Cl, Br, I, C₁₋₄-alkyl, C₆₋₁₀-aryl, C₁₋₄-alkoxy and C₆₋₁₀-aryloxy radicals,
and in which the negative charge of the boronate anion is compensated for by a cation;
(vi) triarylboranes of the formula (III) in which
m is 0,
p is 3,
R² and n are each as defined above;
(vii) tetraarylborates of the formula (III) in which
m is 0,
p is 4,
R² and n are each as defined above,
and in which the negative charge of the boronate anion is compensated for by a cation.

2. Process according to Claim 1, wherein the compound (II) is selected from the group consisting of N-(2-bromo-4-fluorophenyl)acetamide, N-(2-bromophenyl)acetamide, N-(2-bromophenyl)-3-oxobutanamide, N-(2-bromo-4-fluorophenyl)-3-oxobutanamide, 2-bromo-N-(propan-2-ylidene)anilines, 2-bromo-4-fluoro-N-(propan-2-ylidene)aniline, N-(2-bromo-4-fluoro)aniline, N-(2-bromo)aniline.

3. Process according to Claim 1 or 2, wherein the compound of the formula (III) is selected from the group consisting of bis(3,4-dichlorophenyl)borinic acid, bis(2,3-dichlorophenyl)borinic acid, bis(3-dichlorophenyl)borinic acid, bis(4-dichlorophenyl)borinic acid, 4-chlorophenylboronic acid, 3-chlorophenylboronic acid, 2-chlorophenylboronic acid, 3,4-dichlorophenylboronic acid and 2,3-dichlorophenylboronic acid.

4. Process according to any of Claims 1 to 3, wherein the base is selected from alkali metal hydroxides, alkali metal carbonates and alkali metal hydrogencarbonates.

5. Process according to any of Claims 1 to 4, wherein the palladium catalyst a) according to Claim 1 is a complex of palladium in the 0 oxidation state and a phosphine ligand of the formula (V) or a salt thereof.

6. Process according to any of Claims 1 to 4, wherein a palladium catalyst b) according to Claim 1 is used.

7. Process according to any of Claims 1 to 4, wherein a palladium catalyst c) according to Claim 1 is used, and this palladium catalyst c) consists of metallic palladium on activated carbon in the presence of a phosphine ligand of the general formula (V) or a salt thereof.

8. Process according to Claim 6, wherein the salt of the palladium catalyst b) is selected from the group consisting of palladium chloride, palladium acetates, palladium acetylacetonate and bis(acetonitrile)palladium chlorides.

9. Process according to any of Claims 1 to 8, wherein the phosphine ligand of the general formula (V) is selected from di(tert-butyl)phenylphosphine, di-tert-butyl-p-[4-(trifluoromethyl)phenyl]phosphine, 4-(di-tert-butylphosphino)-p-N,N-dimethylaniline and di-tert-butyl-p-(4-methylphenyl)phosphine.

10. Process according to any of Claims 1 to 4, 6 or 8, wherein a palladium catalyst b) is used, where the molar ratio of the palladium salt to the phosphine ligand of the general formula (V) or a salt thereof is 1:1 to 1:5.

11. Process according to any of Claims 1 to 10, wherein 0.001 to 1.0 mol% of the palladium catalyst is used, based on the amount of the compound of the formula (II).

12. Process according to any of Claims 1 to 11, wherein the reaction is conducted at a temperature of 20°C to 100°C.

13. Process according to any of Claims 1 to 12, wherein the reaction is conducted in a mixture of water and an organic solvent.

## Revendications

1. Procédé de fabrication de biphénylanilides halogénés de formule (I) 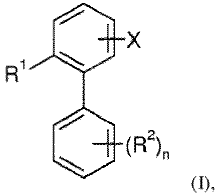 dans laquelle
X est choisi parmi hydrogène, fluor et chlore ;
R¹ est choisi parmi -NH(CO)R³, -N=CR⁴R⁵, NO₂, NH₂ et NHR³ ;
R² représente chlore ;
R³, R⁴, R⁵ sont choisis indépendamment les uns des autres parmi hydrogène, -CH₂-(C=O)CH₃, alkyle en C₁-C₈, alcényle en C₁-C₈, alcynyle en C₁-C₈ et aryle en C₆-C₁₈ ; ou
R⁴, R⁵ peuvent former ensemble avec l'atome de carbone auquel ils sont reliés un cycle à 5 ou 6 chaînons, qui contient 1, 2 ou 3 hétéroatomes choisis parmi N, O et S ;
n est choisi parmi 1, 2 et 3,
par mise en réaction d'un composé de formule (II) 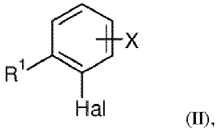 dans laquelle
Hal est choisi parmi brome et iode ; et R¹ et X sont tels que définis précédemment,
en présence d'une base et d'un catalyseur à base de palladium choisi dans le groupe constitué par :
a) un complexe constitué par du palladium à l'état d'oxydation 0 et un ligand phosphine de formule (V) ou un sel de celui-ci,
b) un sel de palladium en présence d'un ligand phosphine de formule (V) ou un sel de celui-ci, et
c) du palladium métallique, éventuellement appliqué sur un support, en présence d'un ligand phosphine de formule (V) ou un sel de celui-ci,
le ligand phosphine de formule (V) étant défini par : 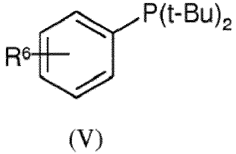 dans laquelle
R⁶ est choisi parmi hydrogène, alkyle en C₁-C₄, haloalkyle en C₁-C₄, phényle et NR⁷, et
R⁷ est choisi parmi (alkyle en C₁-C₄)₂,
ou un sel de celui-ci,
dans un solvant, avec un composé d'organobore de formule (III) 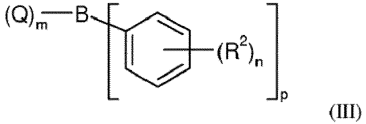
choisi dans le groupe constitué par :
(i) les acides boroniques de formule (III) dans laquelle
m représente 2,
p représente 1,
Q¹ et Q² représentent des groupes hydroxyle,
R² et n sont tels que définis précédemment,
ou les anhydrides, dimères ou trimères formés à partir des acides boroniques de formule (III) ;
(ii) les dérivés d'acide boronique de formule (III), dans laquelle
m représente 2,
p représente 1,
Q¹ et Q² sont choisis chacun indépendamment l'un de l'autre parmi F, Cl, Br, I, les radicaux alkyle en C₁₋₄, aryle en C₆₋₁₀, alcoxy en C₁₋₄ et aryloxy en C₆₋₁₀,
R² et n sont tels que définis précédemment ;
(iii) les acides boriniques de formule (III), dans laquelle
m représente 1,
p représente 2,
Q est choisi parmi OH, F, Cl, Br, I, les radicaux alkyle en C₁₋₄, aryle en C₆₋₁₀, alcoxy en C₁₋₄ et aryloxy en C₆₋₁₀,
R² et n sont tels que définis précédemment ;
(iv) les esters de l'acide boronique cycliques de formule (III), dans laquelle
m représente 2,
p représente 1,
Q¹ et Q² sont chacun choisis indépendamment l'un de l'autre parmi les radicaux alcoxy en C₁₋₄ qui forment ensemble avec l'atome de bore auquel ils sont reliés un cycle à 5 ou 6 chaînons, qui peut être substitué avec des radicaux alkyle en C₁₋₄,
R² et n sont tels que définis précédemment ;
(v) les boronates de formule (III), dans laquelle
m représente 3,
p représente 1,
R² et n sont tels que définis précédemment,
Q¹ à Q³ sont chacun choisis indépendamment les uns des autres parmi OH, F, Cl, Br, I, les radicaux alkyle en C₁₋₄, aryle en C₆₋₁₀, alcoxy en C₁₋₄ et aryloxy en C₆₋₁₀,
et dans laquelle la charge négative de l'anion boronate est compensée par un cation ;
(vi) les triarylboranes de formule (III), dans laquelle
m représente 0,
p représente 3,
R² et n sont tels que définis précédemment ;
(vii) les tétraarylborates de formule (III), dans laquelle
m représente 0,
p représente 4,
R² et n sont tels que définis précédemment,
et dans laquelle la charge négative de l'anion boronate est compensée par un cation.

2. Procédé selon la revendication 1, dans lequel le composé (II) est choisi dans le groupe constitué par le N-(2-bromo-4-fluorophényl)acétamide, le N-(2-bromophényl)acétamide, le N-(2-bromophényl)-3-oxobutanamide, le N-(2-bromo-4-fluorophényl)-3-oxobutanamide, la 2-bromo-N-(propan-2-ylidène)aniline, la 2-bromo-4-fluoro-N-(propan-2-ylidène)aniline, la N-(2-bromo-4-fluoro)aniline, la N-(2-bromo)aniline.

3. Procédé selon la revendication 1 ou 2, dans lequel le composé de formule (III) est choisi dans le groupe constitué par l'acide bis(3,4-dichlorophényl)borinique, l'acide bis(2,3-dichlorophényl)borinique, l'acide bis(3-dichlorophényl)borinique, l'acide bis(4-dichlorophényl)borinique, l'acide 4-chlorophénylboronique, l'acide 3-chlorophénylboronique, l'acide 2-chlorophénylboronique, l'acide 3,4-dichlorophénylboronique et l'acide 2,3-dichlorophénylboronique.

4. Procédé selon l'une quelconque des revendications 1 à 3, dans lequel la base est choisie parmi les hydroxydes de métaux alcalins, les carbonates de métaux alcalins et les hydrogénocarbonates de métaux alcalins.

5. Procédé selon l'une quelconque des revendications 1 à 4, dans lequel le catalyseur à base de palladium a) selon la revendication 1 est un complexe de palladium à l'état d'oxydation 0 et d'un ligand phosphine de formule (V) ou un sel de celui-ci.

6. Procédé selon l'une quelconque des revendications 1 à 4, dans lequel un catalyseur à base de palladium b) selon la revendication 1 est utilisé.

7. Procédé selon l'une quelconque des revendications 1 à 4, dans lequel un catalyseur à base de palladium c) selon la revendication 1 est utilisé et ce catalyseur à base de palladium c) est constitué par du palladium métallique sur du charbon actif en présence d'un ligand phosphine de formule générale (V) ou un sel de celui-ci.

8. Procédé selon la revendication 6, dans lequel le sel du catalyseur à base de palladium b) est choisi dans le groupe constitué par le chlorure de palladium, l'acétate de palladium, l'acétylacétonate de palladium et le chlorure de bisacétonitrile-palladium.

9. Procédé selon l'une quelconque des revendications 1 à 8, dans lequel le ligand phosphine de formule générale (V) est choisi parmi la di(tert.-butyl)phénylphosphine, la di-tert-butyl-p-[4-(trifluorométhyl)phényl]phosphine, la 4-(di-tert-butylphosphino)-p-N,N-diméthylaniline et la di-tert-butyl-p-(4-méthylphényl)phosphine.

10. Procédé selon l'une quelconque des revendications 1 à 4, 6 ou 8, dans lequel un catalyseur à base de palladium b) est utilisé, dans lequel le rapport molaire entre le sel de palladium et le ligand phosphine de formule générale (V) ou un sel de celui-ci est de 1:1 à 1:5.

11. Procédé selon l'une quelconque des revendications 1 à 10, dans lequel 0,001 à 1,0 % en moles du catalyseur à base de palladium est utilisé par rapport à la quantité du composé de formule (II).

12. Procédé selon l'une quelconque des revendications 1 à 11, dans lequel la réaction est réalisée à une température de 20 à 100 °C.

13. Procédé selon l'une quelconque des revendications 1 à 12, dans lequel la réaction est réalisée dans un mélange d'eau et d'un solvant organique.
